# EUROPEAN PATENT APPLICATION

(11) **EP 1 048 636 A1**
(43) Date of publication of application: **02.11.2000**
(21) Application number: 00303198.6
(22) Date of filing: 14.04.2000
(51) Int. Cl.: C07C 17/20, C07C 25/22, C07F 5/02

(54) **Process for mono-brominating a perfluoroaromatic compound**

(30) Priority: 30.04.1999 GB 9909973
(71) Applicant: Laporte Industries Limited, London W1H 9AB (GB)
(72) Inventor: Gardner, Peter Howard, Norton, Stockton-on-Tees TS20 2TW (GB); Heron, Ian James, Harrogate, North Yorkshire HG1 2PB (GB)
(74) Representative: Stuttard, Garry Philip

(57) **Abstract**

A one-step process for preparing 2-bromoheptafluoronaphthalene which is a useful intermediate in the preparation of boron based co-catalysts.

## Description

The present invention relates to a process for the mono-bromination of perfluoroaromatic compounds, in particular for the preparation of 2-bromoheptafluoronaphthalene.

2-bromoheptafluoronaphthalene is useful as an intermediate in the preparation of tris(β-perfluoronaphthyl)boranes which are highly activated co-catalysts for metallocene-activated Ziegler-Natta polymerisation of olefins (see Li *et al*, Organometallics, 1998, 17, 3995-4003). 2-bromoheptafluoronaphthalene is also of value as an intermediate in the synthesis of boron-based co-catalysts for the manufacture of polysilanes (see Newton *et al*, Polym. Prepr. (Am. Chem. Soc; Div. Polym. Chem); 1998, 39(1), 587-58).

Various multi-step processes have been described for the preparation of 2-bromoheptafluoronaphthalene from octafluoronaphthalene. In one process, 2-hydrazinoheptafluoronaphthalene is treated with copper bromide in 48% refluxing hydrobromic acid (see Li *et al*, Organometallics, 1998, 17, 3995-4003 and Brooke *et al*, J. Fluorine Chem, 1990, 50(2), 229-242 and WO-A-99/06412 (Dow Chemical Company)). A 58% yield from the hydrazino compound is obtained by Li *et al* and a 57% yield from the hydrazino compound is obtained by Dow Chemical Company. The 2-hydrazinoheptafluoronaphthalene starting compound may be prepared by treating octafluoronaphthalene with hydrazine hydrate in refluxing ethanol with a yield of about 57.6% to the pure compound (Gething *et al*, J. Chem. Soc., 1962, (36), 186). Thus the overall yield for the first of the known two step processes is approximately 33% from octafluoronaphthalene.

An alternative process involves sequential treatment of 2H-heptafluoronaphthalene with butyl lithium and bromine (see Burdon *et al*, J Chem. Soc. Perkin Trans I, 1980, 11, 2494-6) or with chlorosulphonic acid and bromine (see Shteingarts *et al*, J Org. Chem. USSR (Eng. Trans), 1970, 6, 835-840). The starting compound may be prepared by reduction of the 2-hydrazino- compound or by treatment of octafluoronaphthalene with lithium aluminium hydride (Gething *et al*, J. Org. Chem. 1962, 186-190). Equally the starting compound may be prepared by room temperature hydrogenolysis of perfluorodecalin mediated by a low valent zirconium complex (see Kiplinger *et al*, Chem. Comm., 1996, 10, 1115-1116 and Kiplinger *et al*, J. Amer. Chem. Soc., 1996, 118(7), 1805-1806).

A further route to 2-bromoheptafluoronaphthalene involves the sequential treatment of 2-aminoheptafluoronaphthalene with hydrofluoric acid, sodium nitrite and copper bromide/hydrobromic acid (see Osina *et al*, J Org. Chem. USSR (Eng. Trans), 1980, 16 (4), 706-716).

The present invention seeks to provide an improved process for the mono-bromination of perfluoroaromatics conducted in a single step, in particular an improved process for the preparation of 2-bromoheptafluoronaphthalene in a single step from octafluoronaphthalene.

Thus viewed from one aspect the present invention provides a process for mono-brominating a perfluoroaromatic compound comprising:
reacting said perfluoroaromatic compound with a nucleophilic bromide source in a polar aprotic solvent at an elevated temperature.

The direct (single step) process of the invention is more efficient than known multi-step processes in which a substituted or unsubstituted heptafluoroaromatic compound is prepared and isolated before the primary bromination step. The process has the further advantage over the prior art that it is conducted in the absence of potentially hazardous reagents such as hydrazine.

In a preferred embodiment, the process of the invention is used for preparing 2-bromoheptafluoronaphthalene, said process comprising: reacting octafluoronaphthalene with a nucleophilic bromide source in a polar aprotic solvent at an elevated temperature.

The preferred process of the invention makes possible the direct conversion of octafluoronaphthalene to the 2-bromo-compound in a single step. The process is therefore more efficient than known multi-step processes in which a substituted or unsubstituted heptafluoronaphthalene is prepared and isolated before the primary bromination step.

Other perfluoroaromatic compounds used in preferred embodiments of the process of the invention are unsaturated monocyclic or polycyclic perfluorinated hydrocarbons including *inter alia*:

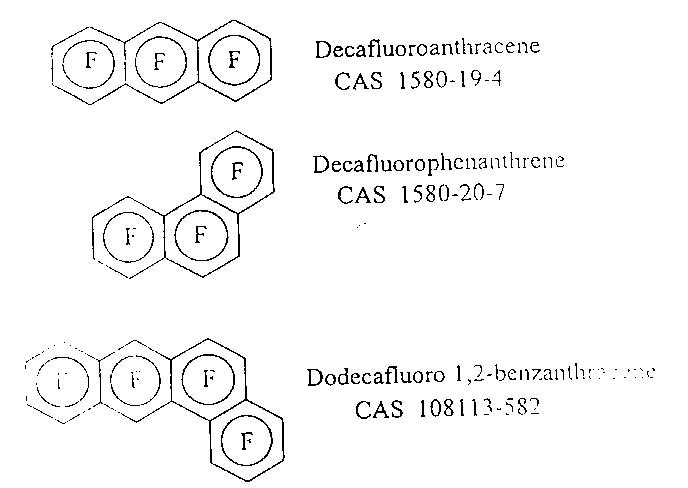

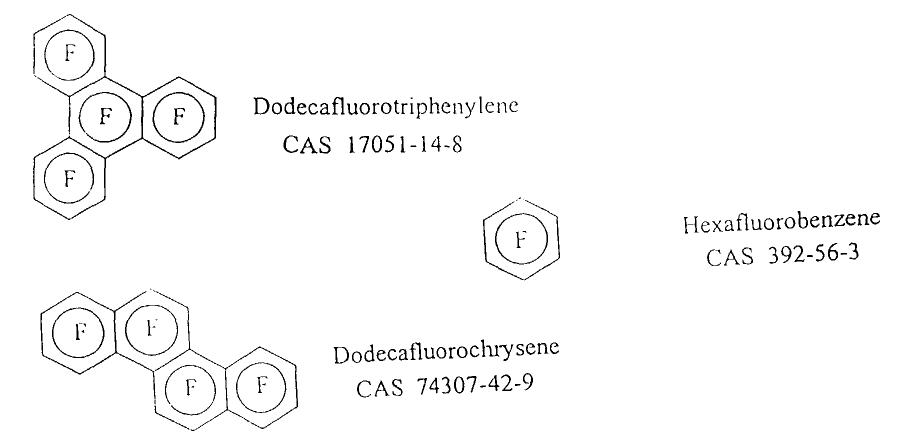

In accordance with the invention, the nucleophilic bromide source is preferably a bromide ion (Br⁻) source. Examples of nucleophilic bromide sources include tetraaryl ammonium bromides, tetraalkyl ammonium bromides, silyl bromides, metal bromides (*eg* alkaline earth metal bromides and alkali metal bromides), tetraalkylphosphonium halides, tetraarylphosphonium halides and polymer supported bromides. Preferred are alkali metal bromides such as sodium, potassium or lithium bromide. Particularly preferred are sodium, potassium and lithium bromide. Especially preferred is lithium bromide.

The reaction may be carried out in one or more polar aprotic solvents such as sulpholane, dimethylformamide, dimethylacetamide, dimethyl sulphoxide, 1,3-dimethylimidazolidinone and N-methyl pyrrolidinone. Sulpholane is a preferred solvent. The reaction may also be carried out in a mixture of two of the above solvents or one of the above solvents together with a hydrocarbon of suitable boiling point (*eg* decalin, tetralin or the like).

The process according to the present invention may be carried out at an elevated temperature *eg* the reflux temperature of the solvent (or solvent mixture) or lower. Typically the reaction temperature will be in the range 150° to 250°C, preferably within the range 180° to 250°C, for example a temperature of about 225°C.

The reaction is conveniently carried out at ambient pressure. Higher pressures may be used where it is, for example, desirable to control the boiling point of the solvent. The reaction time is typically up to 20 hours. The reagents may be conveniently added in a 1:1 molar ratio.

Catalysts such as crown ethers are optionally and advantageously used to improve the process of the invention. Suitable examples include but are not limited to 12-Crown-4, 15-Crown-5, 18-Crown-6, dibenzo-18-crown-6, dicyclohexano-18-Crown-6 (all isomers) and dicyclohexano-24-Crown-8 (all isomers). The preferred catalysts are 18-Crown-6 and dibenzo-18-crown-6.

2-Bromoheptafluoronaphthalene produced in accordance with a preferred embodiment of the invention is useful as an intermediate in the preparation of tris(β-perfluoronaphthyl)boranes which are highly activated co-catalysts for metallocene-activated Ziegler-Natta polymerisation of olefins. 2-Bromoheptafluoronaphthalene may be also be used as an intermediate in the synthesis of boron-based co-catalysts for the manufacture of polysilanes.

Viewed from a further aspect the present invention provides the use of 2-bromoheptafluoronaphthalene prepared by the process as hereinbefore defined for preparing a boron based co-catalyst.

Viewed from a yet further aspect the present invention provides a process for preparing boron based co-catalysts, said process comprising:
reacting octafluoronaphthalene with a nucleophilic bromide source in a polar aprotic solvent at an elevated temperature; optionally isolating at least a portion of the 2-bromoheptafluoronaphthalene product; and
converting the 2-bromoheptafluoronaphthalene product or the isolated portion thereof into a boron based co-catalyst.

The reagents used in accordance with the embodiments of the invention may be available commercially or may be prepared in a conventional manner. For example, octafluoronaphthalene may be prepared in accordance with Fuller, J Chem Soc., 1965, 6264 and Kusov *et al*, Izv. Sib. Otd. Akad. Nauk. SSSR, Ser. Khim. Nauk. 3 (1986), 81. Octachloronaphthalene as a precursor for octafluoronaphthalene may be prepared in accordance with Schwemberger, J Gen. Chem. USSR, 8 (1938), 1353 and Schwemberger *et al*, J Gen. Chem. USSR, 2 (1932), 921.

Unless specified otherwise, references to aryl and alkyl groups hereinbefore are preferably linear or branched C₁₋₆-alkyl and phenyl or substituted phenyl aryl groups.

The invention will now be further described in a non-limitative sense with reference to the following example

### Example

A 250 ml round-bottomed flask was flushed with nitrogen gas and charged with 10g octafluoronaphthalene, 3.2g lithium bromide and 180g sulpholane. The finely divided mixture was heated to 225°C. The composition of the mixture was monitored and showed no increase in the desired 2-bromo product after a period of 20 hours, at which point the mixture was cooled to ambient temperature. The cool mixture was poured into 155ml of water and then subjected to steam distillation yielding approximately 120ml of distillate.

The distillate was extracted three times with 120ml samples of dichloromethane and the extracts were combined and dried over anhydrous magnesium sulphate. After removal of the drying agent, the product was subjected to reduced pressure to remove residual solvent. 8.9g of a white waxy solid product were obtained.

The solid product was analysed and shown to be a mixture of 2-bromoheptafluoronaphthalene and unreacted octafluoronaphthalene in the approximate proportion of 2:1, together with a small quantity of the dibromo-substituted product. Analysis of the crude product by HPLC showed it to comprise approximately 57% area 2-bromo- compound equating to a 45.8% yield.

A portion (3g) of crude product was subjected to purification by flash chromatography. By combining only those fractions which were rich in the desired 2-bromo compound and solvent stripping, 0.43g of pure (98.3% area HPLC) material was obtained. Although the crude product itself may be sufficient for further working, it is expected that the isolation procedure could be further optimised to improve the pure yield.

The solid had a melting point of 70.2 to 70.4°C. The infra-red spectrum of this purified product corresponded to that of the desired 2-bromoheptafluoronaphthalene.

## Claims

1. A process for mono-brominating a perfluoroaromatic compound comprising:
reacting said perfluoroaromatic compound with a nucleophilic bromide source in a polar aprotic solvent at an elevated temperature.

2. A process as claimed in claim 1 wherein the perfluoroaromatic compound is a monocyclic or polycyclic perfluorinated hydrocarbons.

3. A process as claimed in claim 2 wherein the monocyclic or polycyclic perfluorinated hydrocarbons is selected from the group consisting of decafluoroanthracene, decafluorophenanthrene, dodecafluorol,2-benzanthracene, dodecafluorochrysene, hexafluorobenzene and dodecafluorotriphenylene.

4. A process as claimed in any preceding claim for preparing 2-bromoheptafluoronaphthalene, said process comprising:
reacting octafluoronaphthalene with a nucleophilic bromide source in a polar aprotic solvent at an elevated temperature.

5. A process as claimed in any preceding claim wherein the nucleophilic bromide source is a bromide ion (Br⁻)source.

6. A process as claimed in any preceding claim wherein the nucleophilic bromide source is selected from the group consisting of tetraaryl ammonium bromides, tetraalkyl ammonium bromides, silyl bromides, metal bromides, tetraalkylphosphonium halides, tetraarylphosphonium halides and polymer supported bromides.

7. A process as claimed in claim 6 wherein the nucleophilic bromide source is sodium, potassium or lithium bromide.

8. A process as claimed in claim 6 or 7 wherein tie nucleophilic bromide source is lithium bromide.

9. A process as claimed in any preceding claim wherein the polar aprotic solvent is selected from one or more of the group consisting of sulpholane, dimethylformamide, dimethylacetamide, dimethyl sulphoxide, 1,3-dimethylimidazolidinone and N-methyl pyrrolidinone.

10. A process as claimed in claim 9 wherein the polar aprotic solvent is sulpholane.

11. A process as claimed in any preceding claim wherein the polar aprotic solvent is in admixture with a hydrocarbon solvent.

12. A process as claimed in any preceding claim wherein the elevated temperature is the reflux temperature of the solvent or solvent mixture.

13. A process as claimed in any preceding claim wherein the reaction temperature is in the range 150° to 250°C.

14. A process as claimed in any preceding claim wherein the reaction temperature is the range 180° to 250°C

15. A process as claimed in any preceding claim wherein the reaction temperature is about 225°C.

16. A process as claimed in any preceding claim wherein the reaction is conducted in the presence of a crown ether catalyst.

17. A process as claimed in claim 16 wherein the crown ether catalyst is selected from the group consisting of 12-Crown-4, 15-Crown-5, 18-Crown-6, dibenzo-18-crown-6, dicyclohexano-18-Crown-6 (all isomers) and dicyclohexano-24-Crown-8 (all isomers).

18. A process as claimed in either of claims 16 or 17 wherein the catalyst is 18-Crown-6 or dibenzo-18-crown-6.

19. Use of 2-bromoheptafluoronaphthalene prepared by the process as hereinbefore defined for preparing a boron based co-catalyst.

20. A process for preparing boron based co-catalysts, said process comprising:
reacting octafluoronaphthalene with a nucleophilic bromide source in a polar aprotic solvent at an elevated temperature; optionally isolating at least a portion of the 2-bromoheptafluoronaphthalene product; and
converting the 2-bromoheptafluoronaphthalene product or the isolated portion thereof into a boron based co-catalyst.
